(19) Europäisches Patentamt
European Patent Office
Office européen des brevets

(11) Publication number: **0 300 037 B1**

(12) **EUROPEAN PATENT SPECIFICATION**

(45) Date of publication of patent specification: **01.09.93**

(51) Int. Cl.⁵: **G01N 15/14**, G01N 1/30, G01N 33/533

(21) Application number: **88905565.3**

(22) Date of filing: **03.02.88**

(86) International application number:
**PCT/GB88/00065**

(87) International publication number:
**WO 88/05908 (11.08.88 88/18)**

(54) **CELL SCREENING METHODS.**

(30) Priority: **04.02.87 GB 8702441**

(43) Date of publication of application:
**25.01.89 Bulletin 89/04**

(45) Publication of the grant of the patent:
**01.09.93 Bulletin 93/35**

(84) Designated Contracting States:
**AT BE CH DE FR IT LI LU NL SE**

(56) References cited:
US-A- 3 327 119
US-A- 3 916 205
US-A- 4 020 151

Journal of Immmunological Methods, vol.
101, 1987, Elsevier Science Publishers B.V.
(Biomedical Division) R C. Hart et al.: "The
use of acridinium ester-labelled strep-
tavidinin in immunoassays", pages 91-96

Biomedical Engineering, vol. 11, 1976 O.A.N.
Husain et al.: "Automation in cervical cancer
screening - part I: fixed cell scanning sys-
tems", pages 161-166

(73) Proprietor: **RICHMOND CELL SCREENING
LIMITED
152 Bath Street
Glasgow G1(GB)**

(72) Inventor: **SCHOLEFIELD, John
Springbank House Campsie Road
Kirkintilloch Glasgow(GB)**
Inventor: **MILLAR, Ronald, William
7 Kenbank Road Bridge of Weir
Renfrewshire,PA11 3AZ(GB)**

(74) Representative: **Ede, Eric et al
Fitzpatricks 4 West Regent Street
Glasgow G2 1RS (GB)**

Rank Xerox (UK) Business Services
(3.10/3.6/3.3.1)

## Description

This invention relates to methods for the examination of cells, particularly mammalian cells with a view to carrying out screening of specimens automatically. The invention thus finds application in the examination of cells obtained from cervical smears, biopsies, sputa and other body fluids and tissues.

Current methods of examination of particles, cell or chromosome populations generally relate to either:

(a) well established manual microscopy cytological techniques, or

(b) the examination of prepared suspensions using expensive and intricate instrumentation, e.g. 'FACS' (Fluorescence Activated Cell Sorter), 'Cytofluorograph' and 'Coulter'. These systems provide detailed information of the characteristics or classification of a given population, but do not examine a sequence of individual preparations. Since they generally operate by the streaming of a suspension in a liquid laminar flow and/or by passage of the suspension through a narrow orifice (100-200 $\mu$m diam) they are not suitable for the routine examination of native specimens of body fluids or food materials. All operate using a liquid specimen.

(c) image analysis systems have been employed to display, scan and examine standard microscopical preparations of cells or particles. These systems such as 'Quantimet' (Metals Research Limited) or 'Cervifip' employ a video-camera attached to a microscope system which generate image recognition patterns from standard stained microscope slide preparations of normal and abnormal cells. With appropriate microcomputer hardware and software suspect cells can be flagged. However, such systems require the involvement of trained cytologists and pathologists and the system is neither fast nor automated for the pre-screening of specimens for normal and transformed cells.

Known direct fluorescence microscopy (DFM) methods give a high signal noise (S:N) ratio from both abnormal, for example transformed, and normal cells. Thus, it is difficult using this technique to differentiate between normal and abnormal cells when both are present in a specimen. Traditional direct immunofluorescent techniques, for example, fluorescein isothiocyanate (FITC) give low level fluorescent labelling of abnormal cells which provided inadequate S:N ratios for quantitation and signal analysis.

A method for labelling a specimen with an immunofluoresent label is known from R.C. Hart et al.: "The use of acridinium ester labelled streptavidin in immunoassays" in Journal of Immunological Methods, Vol. 101, 1987; pages 91-96; Elsevier Science Publishers (Biomedical Division).

One particular area where an effective automated screening system is required is in cancer screening. There is an urgent need to relieve the burden of specimen examination on gynaecologists, cytologists and other highly qualified staff by providing some means of pre-screening cervical smears, biopsies etc. prior to referral of examinees to clinics for secondary examination by further smears, colposcopy and biopsy examination.

With adequate early and repeat screening and diagnosis it is widely accepted that the incidence of malignant cervical cancer may be reduced.

Accordingly it is an object of this invention to provide for the examination of samples of cells, automatic screening methods and associated reagent kits to meet this problem.

It is a further object of this means to provide the methods and means to facilitate the automatic detection and discrimination of abnormal cells (e.g., transformed) from normal cells.

Accordingly this invention provides means for treating the specimen so that abnormal portions of the specimen will be labelled differently to normal portions of the specimen. More particularly this invention provides a method for labelling a specimen with an immunofluorescent label comprising applying a chromophore to a specimen before applying an immunofluorescent label to said chromophore treated specimen.

This invention also provides a fluorescent reagent for use in such an immunofluorescent assay, said fluorescent reagent comprising streptavidin reacted with an acridinium compound, particularly acridine isothiocyanate.

In the present case specimen preparation may involve taking of smears, biopsies, sputa and other body fluid or tissue samples and preparing monodisperse cell dispersions using the following techniques alone or in combination:-

(i) sonication, (ii) centrifugation (including density gradient centrifugation), (iii) adsorption and desorption, (iv) filtration (including membrane and ultrafiltration), (v) electrophoresis or electrokinesis, (vi) enzyme or chemical treatment, (vii) pH and ionic control, (viii) reverse osmosis. The prepared specimens may be subjected to standard labelling or marking techniques. One of the following methods may be used:-

(a) non-fluorescent standard chromatic staining procedures in either the liquid specimen or following the application of the specimen to the carrier medium, e.g. porous support plastic film, glass plate or film, metal or ceramic or film, membrane or other filter.

(b) direct fluorochrome staining (DFS) by methods known per se, but preferably also including other fluorochrome staining procedures wherein either the surface of the cells or material, including RNA, DNA or chromatin, within the cells are preferentially stained.

(c) direct and indirect immunofluorescent (IF) and immunochromatic staining techniques whereby either (i) fluor-labelled antisera raised to specific cell membrane/cell wall or cytoplasmic nuclear or nucleolar determinants of transformed cells or (ii) biotin-linked antisera raised to monoclonal or polyclonal cell determinants are bound to avidin or streptavidin linked fluor-markers to provide:

- specific discrimination between normal and transformed exfoliate cells in preparations
- the differentiation between CIN 1, 2 and 3 clonogenic and cancerous cells
- the counterstaining of normal or other cells

(d) modifications of differential chromatic and/or fluorescent staining techniques including the periodate-Schiff, thionine-Feulgen, Papanicolaou (PAP).

A preferred method for carrying out the object of the invention is based on methods relying on a modified streptavidin - biotin procedure (SB methods) which allow the use of alternative monoclonal antibodies (MAB), or combinations of MAB's, raised against specific antigenic (e.g. oncogenes, nuclear, nucleolar or cytoplasmic) determinants using only one secondary antibody.

SB methods have been developed for the following determinants:-

(i) anti-RAS - oncogene, nuclear antigen

(ii) anti-P145 - nucleolar antigen

(iii) anticytokeratin - cytoplasmic intermediate filaments

although it is to be understood that the methods described are applicable to the measurements of any determinant.

Preferred methods also utilise initial fixation and pretreatment of the prepared specimen which provides for enhanced contrast by reduction of non-specific fluor-binding with normal cells and background material present in the specimen.

The pretreatment involves the use of chromatic dyes, non-fluorescent stains or negative stains or chemicals which are taken up and combine with normal cells and background material, but which do not affect the desired specific binding with the streptavidin-biotin linked fluor which combines with determined dysplastic cells or portions thereof.

It has been found now that by treating the specimen with a chromophore after it has been fixed but before it is treated with the immunofluorescent label that the signal to noise ratio is greatly improved when the immunofluorescent labelled specimen is examined subsequently. That is to say that treatment of a specimen with a chromophore increases the ratio of the signal from the immunofluorescent label on the abnormal cells to the background noise when compared with a similar specimen not so treated with a chromophore. By chromophore is meant chromatic dye, stain or chemical.

Immunofluorescent labels used to label abnormal cells, for example, cancer cells, will bind to and thus label a specific site in or on the cell. Both abnormal and normal cells may bind immunofluorescent labels at the same specific site in or on the cells to some extent, but may be difficult to discriminate visually between the abnormal cells and normal cells.

It is thought that a chromophore binds to specific sites in or on the normal cell, which were not present in the abnormal cells which the particular test measures, and thus prevents the immunofluorescent label from then binding to those sites in the normal cells, but not from binding to the corresponding sites in the abnormal cells. Thus the amount of immunofluorescent label bound by the normal cells is decreased thus increasing, when expressed as a ratio, the amount of immunofluorescent label bound by the abnormal cells. Thus the sensitivity of the measurements with methods which use chromophores is increased allowing smaller numbers of abnormal cells to be measured. Indeed existing methods of immunofluorescent labelling are not sensitive enough to allow the detection of abnormal cells in many instances.

A chromophore is a chromatic dye, stain or a chemical which binds preferentially to normal cells and background material when compared with its binding to abnormal cells preventing, blocking or reducing the subsequent binding of an immunofluorescent label to the normal cells and background material, but not to the abnormal cells, thus enhancing the ratio of the amount of an immunofluorescent label bound to abnormal cells compared with the amount of an immunofluorescent label bound to normal cells and background material.

A suitable chromophore is iodine, or potassium halide, which bind to glycogen in normal cells, but not to certain cancer cells which lack glycogen. The immunofluorescent label then binds to sites in or on the abnormal cells, but not to sites in or on normal cells because these have been blocked by the chromophore.

Other suitable chromophores include ethanoic acid and bromothymol blue, nigrosine, Indian ink, and Sudan black. For example Sudan black binds to lipids, and ethanoic acid and bromothymol blue bind to proteins.

One preferred labelling method is described below. This method may be used on its own or preferably in conjunction with signal analysis apparatus. In a particularly preferred embodiment the method described below gives particularly favourable results because it enables the detection and recordal of the initial transitory (duration of about 2 - 3 nanoseconds) increase in fluorescence from the immunofluorescent labelled abnormal cells on irradiation. After this transitory increase the fluorescence decreases to a lower level. Thus measuring this initial transitory high level of fluorescence from the immunofluorescent labelled abnormal cells, increases the sensitivity of the tests.

An example of one preferred labelling method (SB method).

Cervical Smear Evaluation
Procedure

Stage (1)

A unit quantity of cervical smear is applied to a unit area of a solid or porous substrate carrier, e.g. 1.0 x 2.0 cm or 1.5 to 2.0 cm diameter.

Stage (2)

The specimen is dried at 25°C by passage of air over or through the substrate carrier and is immediately subjected to chemical fixation. Suitable chemical fixators include propan-2-ol, ethanol.

Stage (3)

The fixed specimen and carrier is subjected to a solution or solutions of chromophore. Suitable chromophores include iodine, or potassium halide solutions, ethanoic acid, bromothymol blue or other chromatic stain. The specimen and carrier are then rinsed in an isotonic buffered reagent at pH 7.2.

Stage (4)

The fixed specimen and carrier is subjected to a selected antiserum, or antisera, but not necessarily an enzyme-linked antiserum, or antisera. The antiserum can be applied as either a liquid or aerosol or in a form adsorbed on a porous matrix placed on, passed through or in contact with the fixed specimen and carrier. Suitable antiserum include anticytokeratin, anti-RAS and anti-P145. The reaction is allowed to continue for a defined period, suitably 15 mins at a defined temperature, suitably 37°C before removal of the reagent. The specimen and carrier are then rinsed in an isotonic buffer reagent at pH 7.2.

Stage (5)

The fixed specimen is then held in the manner described in (4) with a reagent incorporating an anti-species specific, suitably anti-mouse, biotinilated IgG (Immunoglobulin G) for a defined period, suitably 10 mins and at a defined temperature, suitably 37°C. The specimen and carrier are then rinsed in an isotonic buffered reagent at pH 7.2.

Stage (6)

The specimen and carrier is held as described in (4) and subjected to a reagent containing streptavidin and a label fluor. Suitable label fluors include an acridinium stain, Texas red, phycobiliprotein. The specimen and carrier and the reagent are held for a defined period, suitably 10 mins at a defined temperature, suitably 37°C.

4

Stage (7)

The fixed and processed specimen and carrier are then rinsed in isotonic buffered reagent at pH 7.2 containing an additive prior to examination. Suitable additives include 0.2% (w/v) glycerol.

The object of these methods is to provide prepared specimens which will generate signals which may be detected and used to discriminate between normal and abnormal cells (or parts of cells) or portions of the specimen. By portions of the specimen it is meant to include, without limitation, cells, tissues, and parts thereof.

It has been further found that label fluors of the acridinium type provide an enhanced fluorescence when compared with conventional fluors, for example FITC. A preferred acridinium fluor is acridine isothiocyanate (AITC). The acridinium fluors, for example, AITC, is reacted with streptavidin and the resulting compound is then reacted with the specimen which has been treated with anti-species specific biotinilated IgG. The resultant fluorescence, when the specimen is irradiated and examined, is greater than that which would be obtained using streptavidin reacted with a fluor other than an acridinium compound. This enhanced fluorescence with streptavidin and an acridinium compound fluor increases further the sensitivity of the methods described herein.

The modular nature of the methods described herein allows the labelling of several markers, in abnormal cells, simultaneously using specific xanthoantisera for each determinant coupled to different fluorochromes. In a further embodiment the reagents, normally in a liquid form, may be absorbed or adsorbed, for example, onto inert carriers before being brought into contact with the specimen.

Preferably the specimen is maintained at a temperature of about 37°C at least at each stage of treatment, the duration of which is preferably from 0.5 to 20 minutes.

The specimens would be treated either as liquid specimens (cell suspensions) or when fixed to a suitable support such as a microporous carrier, glass plate, glass fibre, metal or ceramic plate or film, membrane or other filter surface, or alternatively a multiple well specimen test plate of square, rectangular or circular shape may be used. A preferred support is a microporous carrier such as a Nucleopore (Trade Mark) filter or a microporous polycarbonate filter. By microporous carrier is meant a carrier which has pores, holes, voids or spaces in or through it of such a size that the cells in the specimen cannot penetrate the carrier, rather they are retained on or near the surface of the microporous carrier, whilst liquids and reagents are free to move through the structure of the microporous carrier. The carrier or support chosen may in each case be precoated with a single or combination of reagents including binders, adherence improvers, fluors or xanthoantisera (labelled or unlabelled with fluor or chromatic stain) against transformed (neoplastic) determinants expressed within or on the surface of cancer cells.

According to another aspect of the invention there is provided a method of differentiating cells comprising the steps of taking a specimen, preparing a monodisperse cell suspension of said specimen by techniques known per se, and subsequently in an automatic sequence applying the prepared specimen to a support, fixing the applied specimen, labelling the specimen with a determinable marker reagent, passing the labelled specimen to an automatic detector capable of scanning the specimen and registering presence or absence of labelled cells to produce a signal which may be converted to analytical data in a microprocessor unit.

In an alternative method of this invention the specimen is labelled prior to application to the specimen support. Thus, for example, the cell suspension may be treated with a staining reagent.

Thus for carrying out the methods of the invention, there is provided a reagent kit comprising a specimen support, preferably precoated with a specimen adherence improver or binder, a fixative solution, a labelling solution containing a determinable marker and where necessary appropriate buffer and wash solutions.

The following Example illustrates a method of this invention.

Specific example of Protocol SB3 (Streptavidin-Biotin)

1. Prepare standard smear of cells, or specimen, dry with air at 25°C and fix with ethanol.
2. Add 100 μl 1.0% w/v aqu. iodine as chromophore for 30 secs at 25°C.
3. Remove reagent with phosphate saline buffer pH 7.2 rinse
4. Add 50 μl murine monoclonal determinant and react for 15 mins at 37°C
5. Remove reagent with phosphate saline buffer pH 7.2 rinse.
6. Add 50 μl antimouse biotinilated IgG and react for 10 mins at 37°C.
7. Remove reagent with phosphate saline buffer pH 7.2 rinse.
8. Add 50 μl streptavidin-fluor complex and react for 10 mins at 37°C.

9. Remove reagent with phosphate saline buffer pH 7.2 rinse.

10. Examine using an apparatus such as described herein.

Using Protocol SB3a reference dysplastic (abnormal) and normal cell strains and positive and negative specimens provided the following results:-

## Signal : Noise Ratios

| Cell type | Microamperes per micron diameter of cell | | Nanoamperes per cell | |
|---|---|---|---|---|
| Dysplastic/Positive | mean | SD | mean | SD |
| | 3.4 | 0.72 | 890 | 126 |
| Normal/Negative | 1.17 | 0.63 | 243 | 79 |

In addition the SB family of Protocols allows the expression of a Degree of Transformation (Dysplasia DOT) for a cell population or specimen cells.

In the case of cervical cancer screening this DOT has been found to relate to the traditional grading of Cervical Intraepithelial Neoplastic (CIN) cells in smears prepared by reference cytological (Papanicolaou) staining procedures.

## Claims

1. A method for labelling a specimen with an immunofluorescent label characterised by first applying a chromophore to the specimen and then applying an immunofluorescent label to the chromophore treated specimen.

2. A method as claimed in claim 1 in which the chromophore is selected from iodine, potassium halide, ethanoic acid and bromothymol blue.

3. A method as claimed in claim 1 or claim 2 wherein the specimen is initially applied and fixed to a support before application of the chromophore.

4. A method for labelling a specimen with an immunofluorescent label as claimed in claim 3 wherein the immunofluorescent label is applied to said fixed specimen after chromophore treatment in three stages, the first stage comprising treatment with an antiserum, the second stage comprising treatment with an anti-species specific biotinilated IgG and the third stage comprising treatment with a fluorescent reagent.

5. A method for labelling a specimen with an immunofluorescent label characterised by applying and fixing the specimen to a support, applying a chromophore to said fixed specimen, removing excess chromophore from said fixed specimen, applying an antiserum, removing excess antiserum, applying an anti-species specific biotinilated IgG, applying fluorescent reagent, removing excess fluorescent reagent, irradiating said treated specimen and scanning said irradiated specimen to determine the portion of the specimen which has been labelled.

6. A method as claimed in any one of claims 1 to 5 in which the fluorescent reagent is streptavidin reacted with an acridinium compound.

7. A method as claimed in any one of claim 6 in which the fluorescent reagent is streptavidin reacted with acridine isothiocyanate.

8. A fluorescent reagent for use in an immunofluorescent assay as claimed any one of claims 1 to 7 wherein said fluorescent reagent comprises streptavidin reacted with acridine isothiocyanate.

6

## Patentansprüche

1. Verfahren zum Markieren einer Probe mit einem Immunfluoreszens-Marker, dadurch gekennzeichnet daß zunächst ein Chromophor zu der Probe hinzugegeben wird und sodann ein Immunfluoreszens-Marker zu der mit dem Chromophor behandelten Probe hinzugegeben wird.

2. Verfahren nach Anspruch 1, bei dem der Chromophor aus den Stoffen Jod, Kaliumhalogenid, Essigsäure oder Bromothymolblau augewählt ist.

3. Verfahren nach den Ansprüchen 1 oder 2, bei dem die Probe vor der Zugabe des Chromophors zunächst auf einen Träger aufgegeben und dort fixiert wurde.

4. Verfahren zum Markieren einer Probe mit einem Immunfuoreszens-Marker, nach Anspruch 3, bei dem der Immunfluoreszens-Marker zu der fixierten Probe in drei Stufen nach der Chromophorbehandlung hinzugegeben wird, wobei die erste Stufe die Behandlung mit einem Antiserum umfasst, die zweite Stufe die Behandlung mit abtispezies-spezifischem, biotinyliertem IgG umfasst und die dritte Stufe die Behandlung mit einem Fluoreszensagens umfasst.

5. Verfahren zum Markieren einer Probe mit einem Immunfluoreszens-Marker, dadurch gekennzeichnet, daß eine Probe auf einen Träger gegeben und dort fixiert wird, ein Chromophor der fixierten Probe zugegeben wird, der Überschuß an Chromophor von der fixierten Probe entfernt wird, ein Antiserum hinzugegeben wird, der Überschuß an Antiserum entfernt wird, ein antispezies-spezifisches, biotinyliertes IgG zugegeben wird, ein Fluoreszensagens zugegeben wird, der Überschuß an Fluoreszensagens entfernt wird, die behandelte Probe beleuchtet wird und die beleuchtete Probe durchgemustert wird, um den Teil der Probe zu finden, der markiert wurde.

6. Verfahren nach einem der Ansprüche 1 bis 5, bei dem das Fluoreszensagens Strepavidin ist, das zuvor mit einer Acridininverbindung reagiert hat.

7. Verfahren nach Anspruche 6, bei dem das Fluoreszensagens ein Strepavidin ist, das zuvor mit Acridinisothiocyanat ragiert hat.

8. Fluoreszensagens zur Verwendung bei einem Immunofluoreszenz-Test nach einem der Ansprüche 1 bis 7, dadurch gekennzeichnet, daß es Strepavidin enthält, das zuvor mit Acridinisothiocyanat reagiert hat.

## Revendications

1. Procédé pour marquer un échantillon avec une marque immunofluorescente, caractérisé en ce qu'on applique tout d'abord un chromophore à l'échantillon, puis en ce qu'on applique une marque immunofluorescente à l'échantillon traité au chromophore.

2. Procédé selon la revendication 1 dans lequel le chromophore est choisi parmi l'iode, un halogénure de potassium, l'acide éthanoïque et le bleu de bromothymol.

3. Procédé selon la revendication 1 ou la revendication 2 dans lequel l'échantillon est initialement appliqué et fixé à un support avant application du chromophore.

4. Procédé pour marquer un échantillon avec une marque immunofluorescente selon la revendication 3, dans lequel la marque immunofluorescente est appliquée audit échantillon fixé après traitement au chromophore en trois étapes, la première étape comprenant le traitement avec un antisérum, la seconde étape comprenant le traitement avec une IgG biotinilée spécifique contre les espèces, et la troisième étape comprenant le traitement avec un réactif fluorescent.

5. Procédé pour marquer un échantillon avec une marque immunofluorescent, caractérisé en ce qu'on applique et en ce qu'on fixe l'échantillon à un support, en ce qu'on applique un chromophore audit échantillon fixé, en ce qu'on enlève l'excès de chromophore dudit échantillon fixé, en ce qu'on applique un antisérum, en ce qu'on enlève l'excès d'antisérum, en ce qu'on applique une IgG biotinilée spécifique contre les espèces, en ce qu'on applique un réactif fluorescent, en ce qu'on enlève l'excès de réactif fluorescent, en ce qu'on irradie ledit échantillon traité et en ce qu'on effectue un balayage sur ledit échantillon traité pour déterminer la partie de l'échantillon qui a été marquée.

6. Procédé selon l'une quelconque des revendications 1 à 5 dans lequel le réactif fluorescent est la streptavidine que l'on fait réagir avec un composé d'acridinium.

7. Procédé selon l'une quelconque des revendications 1 à 6 dans lequel le réactif fluorescent est la streptavidine que l'on fait réagir avec l'isothiocyanate d'acridine.

8. Réactif fluorescent pour utilisation dans un dosage d'immunofluorescence selon l'une quelconque des revendications 1 à 7, ledit réactif fluorescent comprenant de la streptavidine que l'on fait réagir avec de l'isothiocyanante d'acridine.